# FASCICULE DE BREVET EUROPEEN

(11) **EP 3 474 015 B1**
(45) Date de publication et mention de la délivrance du brevet: **24.06.2026**
(21) Numéro de dépôt: 17196896.9
(22) Date de dépôt: 17.10.2017
(51) Int. Cl.: G01N 33/68

(54) **UTILISATION DE LA PROTÉINE S100B COMME BIOMARQUEUR POUR LA DÉTECTION D'UN RISQUE DE LÉSION CÉRÉBRALE LORS D'UN ACCOUCHEMENT CONSIDÉRÉ COMME NORMAL**
VERWENDUNG DES PROTEINS S100B ALS BIOMARKER ZUR ERKENNUNG EINES RISIKOS FÜR GEHIRNSCHÄDEN BEI EINER ALS NORMAL ANGESEHENEN GEBURT
USE OF S100B PROTEIN AS A BIOMARKER FOR THE DETECTION OF A RISK OF BRAIN INJURY DURING NORMAL CHILDBIRTH

(43) Date de publication de la demande: 24.04.2019
(73) Titulaire: Ami, Olivier, 75116 Paris (FR); Fauck, Denis, 95110 Sannois (FR)
(72) Inventeur: Ami, Olivier, 75116 Paris (FR); Fauck, Denis, 95110 Sannois (FR)
(74) Mandataire: Demulsant, Xavier

(56) Documents cités:
- LIANG W. ET AL.: "Obstetric conditions and risk of first ever mental health contact during infancy, childhood and adolescence", MIDWIFERY, vol. 28, no. 4, 2012, pages 439 - 444, XP028930347
- SATRIANO A. ET AL.: "The potentials and limitations of neuro-biomarkers as predictors of outcome in neonates with birth asphyxia", EARL. HUM. DEV., vol. 105, 18 December 2016 (2016-12-18), pages 63 - 67, XP029922426
- HONGYAN L. ET AL.: "Neonatal hypoxic ischemic encephalopathy-related biomarkers in serum and cerebrospinal fluid", CLIN. CHIM. ACTA, vol. 450, 28 August 2015 (2015-08-28), pages 282 - 297, XP029297207
- QIAN J. ET AL.: "Umbilical artery blood S100Î protein: a tool for the early identification of neonatal hypoxic-ischemic encephalopathy", EUR. J. PEDIATR., vol. 168, no. 1, 9 April 2008 (2008-04-09), pages 71 - 77, XP019658205
- WIRDS J.W. ET AL.: "S100 protein content of umbilical cord blood in healthy newborns in relation to mode of delivery", ARCH. DIS. CHILD. FETAL NEONATAL ED., vol. 88, no. 1, January 2003 (2003-01-01), pages F67 - F69, XP055700247
- AMI OLIVIER: "Protéine S100B et accouchement : un nouveau marqueur de bien-être cérébral foetal", 2 January 2017 (2017-01-02), XP55949985, Retrieved from the Internet <URL:https://fr.linkedin.com/pulse/prot%C3%A9ine-s100b-et-accouchement-un-nouveau-marqueur-de-ami-olivier?trk=portfolio_article-card_title>

## Description

L'invention a trait au domaine de l'obstétrique, plus particulièrement de l'accouchement.

L'invention trouve une application particulièrement avantageuse dans l'identification de risques de lésions cérébrales, lors d'accouchements considérés comme normaux, chez des nouveau-nés asymptomatiques, en l'absence de souffrance fœtale.

Par « *lésion cérébrale* » on désigne ici les hémorragies, les œdèmes et ischémies cérébrales.

Par « *accouchement* » on désigne ici l'ensemble des phénomènes qui conduisent à l'expulsion du fœtus et des annexes (placenta, liquide amniotique et membranes). Lors de l'accouchement, est appelée présentation la partie du fœtus qui occupe l'aire du détroit supérieur pour s'y engager et évoluer ensuite suivant un mécanisme qui lui est propre.

Par « *accouchement normal* », on désigne ici un accouchement eutocique, qui aboutit par la seule influence des phénomènes naturels à l'expulsion de l'enfant par voie basse, hors des voies génitales maternelles, la grossesse ayant atteint le terme théorique de 28 semaines d'aménorrhée. Le déroulement d'un accouchement normal comprend des périodes portant le nom de travail.

Dans un accouchement normal, la souffrance fœtale est considérée comme sans impact sur l'enfant.

Par « *souffrance fœtale* » on désigne ici un état pathologique du fœtus par hypoxie ou par alimentation insuffisante.

Par « *nouveau-né asymptomatique* » on désigne ici un nouveau-né ne présentant aucun signe d'acidose ou d'hypoxie, et présentant notamment un score d'Apgar normal, un pH de sang ombilical normal.

Par sa définition même, un accouchement normal n'est déclaré qu'après la naissance, au vu des données rétrospectives de la marche du travail et de l'état du nouveau-né.

Un accouchement normal est opposé à un accouchement dystocique, qui entraîne des difficultés ou des impossibilités d'accouchement par voie basse. Les dystocies peuvent résulter d'une présentation délicate, par exemple présentation de l'épaule, présentation du siège. Les dystocies peuvent également résulter d'anomalies de la contraction utérine et de la dilatation du col, ou être d'origine osseuse (bassins rétrécis symétriques ou asymétriques), voire d'origine fœtale (hydrocéphalie, fœtus de grand volume). Les présentations céphaliques sont les plus fréquentes, de l'ordre de 95% des cas, et regroupent la présentation du sommet (dans laquelle l'occiput est la partie de la tête qui descend la première), la présentation de la face (dans laquelle la face toute entière, menton compris, est la partie de la tête qui descend la première), et la présentation du front, intermédiaire entre la présentation du sommet et celle de la face. Les présentations du siège sont rares, de l'ordre de 4% des naissances. Il en va de même des présentations transversales et obliques (présentations de l'épaule).

Dans un accouchement normal, sous l'effet des contractions utérines, le mobile fœtal va franchir les étages de la filière pelvi-génitale (détroit supérieur, excavation pelvienne, détroit inférieur, périnée).

Un accouchement normal est opposé à un accouchement mettant en œuvre une extraction instrumentale, à l'aide de forceps (par exemple forceps de Tarnier, de Pajot, ou de Demelin-Suzor), de spatules (par exemple spatule de Thierry), de ventouses obstétricales, ou d'un dispositif d'Odon. Même exceptionnelles, les lésions fœtales par suite de l'utilisation de ces extracteurs sont connues, et notamment les fractures du crâne et lésions cérébro-méningées, lors de manœuvres difficiles ou brutales de forceps, ou lorsque la traction sur la ventouse n'est pas perpendiculaire à l'axe de la cupule.

Dans un accouchement normal, une première période de travail conduit à la dilatation du col de l'utérus. Cette première étape est marquée par l'apparition des contractions utérines. Une deuxième étape va de cette dilatation à l'expulsion. Après naissance de l'enfant intervient la troisième étape, jusqu'à expulsion du placenta (délivrance), suivie de la quatrième étape allant jusqu'à la stabilisation des constantes maternelles.

La deuxième étape du travail commence par l'engagement, c'est-à-dire le franchissement du détroit supérieur par le plus grand diamètre de la présentation. L'engagement est suivi de la descente et de la rotation de la tête fœtale dans l'excavation pelvienne, puis de l'expulsion. Il est généralement accepté que l'accommodation de la tête fœtale au détroit supérieur comprend quatre mécanismes : une flexion céphalique, une orientation en oblique de la tête fœtale, une inclinaison latérale ou asynclitisme, et des déformations plastiques de la tête fœtale (occipito-antérieures, occipito-postérieures, front et face), ces quatre mécanismes intervenant quasi simultanément et n'étant pas tous observés : l'asynclitisme et les déformations plastiques de la tête sont facultatifs dans les accouchements normaux. Les déformations osseuses crâniennes par chevauchement des os ou par modifications de leurs courbures peuvent intervenir lorsque le travail a été long, et se rencontrent souvent dans les accouchements dystociques.

Le développement de l'imagerie anténatale et néonatale, la mise au point de diagnostics anténataux et de méthodes de monitoring lors du travail, la généralisation des techniques d'analgésie péridurale, la réduction du nombre de maternités et la concentration des moyens matériels et du savoir-faire qui en résultent (cf. décret de périnatalité en France), la part croissante des naissances par césariennes, le développement des techniques de procréation médicalement assistée (PMA) ont modifié les attentes des parents.

L'accouchement est vu par les parents comme un acte très encadré par une équipe médicale, avec des risques qui doivent être identifiés et quantifiés.

Or, le déroulement d'un accouchement n'est actuellement pas parfaitement prédictible, et l'épreuve de travail est fréquente.

Dans le même temps, l'amélioration du statut social de la femme et de son niveau d'éducation sont associés à un nombre d'enfants par famille en baisse, et à un âge de la mère au premier enfant en augmentation. Par exemple, selon l'INSEE, l'âge moyen de la mère, au premier enfant, était en France de 26 ans en 1975 et de 30.6 ans en 2015.

L'accouchement est ainsi de plus en plus une expérience unique pour les parents.

L'application du principe de précaution et l'obligation pour le praticien de respecter la volonté de la personne, après l'avoir informée des conséquences de son choix et de leur gravité (article L 1111-4 du code de la santé publique français) pourraient amener à une augmentation du nombre de césariennes programmées, en raison de la crainte d'un éventuel recours en justice en cas d'accouchement par les voies naturelles avec complication, ou de souffrances fœtales avant mise en œuvre d'une césarienne en urgence.

Il est en effet tentant de recourir aux techniques les plus médicalisées, présentant a priori les conditions de contrôle les plus strictes, en particulier la césarienne. En 1962, le taux de césarienne était en France de 6%, et de 21% en 2010, la moitié des césariennes étant réalisées avant tout travail. Avec plus de 200 000 cas par an, la césarienne est aujourd'hui en France l'intervention chirurgicale la plus fréquente. Selon les données de l'OMS et de l'OCDE, les taux de césarienne seraient de 31% aux Etats Unis, de 36% au Brésil, de 49% au Mexique, et ce taux serait de 52% en Chine, dans le cadre de la politique de l'enfant unique. Les césariennes peuvent résulter d'indications médicales (par exemple en cas d'utérus cicatriciel, de grossesse multiple, la mise en œuvre d'une césarienne prophylactique étant décidée en fonction de la valeur du diamètre bipariétal et de l'indice de Magnin), mais les césariennes résultent dans un nombre important de cas, d'une demande de la mère.

Dans ce contexte technique, médical, sociétal et légal, des efforts importants ont été mis en œuvre depuis des décennies pour détecter la souffrance fœtale et évaluer l'état de santé du nouveau-né, en particulier les risques de retard et d'infirmité motrice ou cérébrale, consécutifs à une souffrance fœtale aigüe.

Avant l'accouchement, les risques d'hypoxie peuvent être identifiés par une prise en compte des facteurs de risques liés à l'état de santé de la mère (notamment anémie, diabète, grossesse prolongée, hypotension iatrogène, insuffisance respiratoire), à l'état du placenta (notamment placenta prævia), à l'aspect du cordon, ainsi que par la prise en compte de certaines caractéristiques du fœtus (prématurité, incompatibilité rhésus, cardiopathie congénitale, gros enfant, grossesse gémellaire). L'hypoxie prepartum peut être également d'origine infectieuse (bactérienne ou amniotite), ou d'origine traumatique (notamment en cas d'épreuve du travail prolongée, dystocie dynamique, présentation dystocique).

Pendant le travail, l'état du fœtus est classiquement surveillé par différentes méthodes :
- contrôle de l'aspect du liquide amniotique, par amnioscopie,
- enregistrement du rythme cardiaque fœtal (RCF) pendant toute la phase active du travail (cardiotocographe, capteur à effet Doppler, à travers la paroi abdominale, ECG par une électrode au scalp),
- mesure du pH sanguin capillaire par micro-prélèvement à partir du scalp du fœtus,
- mesure des lactates au scalp,
- oxymétrie transcutanée par voie vaginale, en plaçant un capteur sur le scalp d'un enfant à naître ou sur sa joue.

L'amnioscopie n'est plus systématique chez les femmes entrant en salle de naissance, en l'absence de démonstration de l'utilité de cet examen. La détection de liquide méconial dans le liquide amniotique est un signal d'alerte, mais n'est pas spécifique.

L'enregistrement du RCF permet une analyse rétrospective et d'éventuelles expertises médico-légales. L'interprétation des anomalies du RCF présente toutefois de nombreux inconvénients.

L'une des difficultés de l'interprétation du RCF est la définition de critères de normalité du rythme cardiaque fœtal, proposés par les sociétés savantes, avec des classifications des RCF différentes suivants les pays (*International Federation of Gynecology and Obstetrics FIGO, National Institute of Child Health and Human Development NICHD, Royal College of Obstretricians and Gyneacologists RCOG, Society of Obstetricians and Gynecologists of Canada SOGC, Agence Nationale d'Accréditation et d'Evaluation en Santé ANAES, American College of Obstetrician and Gynecologists ACOG, Collège National des Gynécologues et Obstétriciens Français CNGOF*)*.* Il est conventionnellement accepté en France que le rythme de base doit être compris entre 110 et 160 battements par minutes (bpm), avec une variabilité comprise entre 6 et 25 bpm, avec absence de ralentissements. Le CNGOF dans ses recommandations de pratique clinique définit ainsi des rythmes normaux, à faible risque d'acidose, à risque d'acidose, à risque important d'acidose et à risque majeur d'acidose.

Une autre difficulté de l'interprétation du RCF est la variabilité inter et intra-observateur. La RCF a une faible spécificité avec un taux de faux positif élevé d'environ 30% et n'est pas un bon examen de diagnostic. La proportion élevée de faux positif provoque un recours inutile aux césariennes et aux extractions instrumentales.

L'analyse du pH fœtal par microponction du scalp, décrite dès 1962, est considérée comme une méthode de référence pour diagnostiquer une asphyxie pendant le travail, en cas d'anomalie du rythme cardiaque fœtal. Le pH au scalp est une méthode de deuxième ligne couramment utilisée. L'analyse du pH fœtal présente toutefois de nombreux inconvénients. Cet examen est invasif et les taux d'échec de mesure sont non négligeables (notamment par suite d'un volume de sang insuffisant, ou d'une contamination par le liquide amniotique). De plus, le pH fœtal peut diminuer rapidement, le résultat de la mesure n'étant qu'une valeur instantanée.

La mesure des lactates au scalp est semblable à la mesure de pH, mais demande une quantité de sang moindre. La mesure des lactates présente l'inconvénient de ne permettre qu'une surveillance discontinue.

L'oxymétrie est délicate à mettre en œuvre, en particulier pour maintenir un contact permanent du capteur avec la tempe ou la joue fœtale, en présence de liquide amniotique, voire de vernix ou méconium.

Lorsque l'accouchement est terminé, une acidose avec une concentration de lactates de l'ordre de 10 mmol/L ou plus et/ou un score Apgar inférieur à 7 à 5 minutes sont considérés comme symptomatiques d'une asphyxie prénatale.

L'hypoxie entraîne une acidose, et un ralentissement du rythme cardiaque fœtal, ainsi qu'une expulsion de méconium dans le liquide amniotique, et des troubles vasomoteurs cérébraux responsables d'ischémie et d'hémorragies cérébro-méningées.

L'hypoxie prepartum peut conduire à une hypoxie - ischémie cérébrale, pouvant conduire à une encéphalopathie néonatale, avec des risques de séquelles, certains enfants devenant infirmes moteurs cérébraux.

En cas d'hypoxie, une encéphalopathie néonatale peut ainsi advenir, dans la première semaine, secondaire à un œdème avec hypertension cérébrale, associant de manière variable des signes de dépression du système nerveux central, des signes d'atteinte des voies motrices corticales, sous corticales et spinales, et des signes d'atteinte du tronc cérébral.

L'imagerie cérébrale du nouveau-né ne permet pas d'affirmer de manière constante la nature anoxo-ischémique des images observées.

L'échographie permet de repérer les pathologies hémorragiques, en cas d'encéphalopathie néonatale, mais ne permet pas une datation de l'évènement hypoxique. L'échographie transfontanellaire est de première intention chez le nouveau-né ayant une encéphalopathie néonatale, à la recherche d'une cause telle qu'une hémorragie ou un hématome sous dural post-traumatique. En dehors de ces rares cas, l'apport de cette technique d'imagerie est considéré comme limité.

Le scanner cérébral est indiqué en urgence en cas de suspicion de traumatisme ou d'hémorragie intracrânienne à l'échographie.

L'imagerie par résonance magnétique est de référence dans l'appréciation pronostique des encéphalopathies asphyxiantes. L'interprétation de cet examen nécessite toutefois une expérience en neuroradiologie néonatale.

Lorsqu'une hypoxie ou une anomalie a été détectée chez le nouveau-né, différents marqueurs biologiques ont été proposés pour les risques d'apparition de déficits cérébraux.

Dans une revue publiée en 2017, Satriano et al présentent différents biomarqueurs envisagés (activine A, protéine acide fibrillaire gliale GFAP, énolase neuro spécifique NSE, protéine S100B, adrénomédulline AM), au stade de recherche clinique, comme prédicteurs de déficits cérébraux chez les nouveaux nés présentant une hypoxie (The potentials and limitations of neuro-biomarkers as predictors of outcome in neonates with birth asphyxia, Early Human Development 105, 2017, pp. 63-67*).*

Dans une revue publiée en 2015, Bersani et al présentent différents biomarqueurs envisagés (protéine S100B, adrénomédulline AM, érythropoïétine, activine A, énolase neuro spécifique NSE, protéine acide fibrillaire gliale GFAP, isoenzyme de la créatine kinase CKBB), au stade de recherche clinique, comme prédicteurs de dommage cérébral néonatal (Use of early biomarkers in neonatal brain damage and sepsis : state of the art and future perspectives, BioMed Reaseach International, 2015, article 253520*).*

Une méta-analyse plus ancienne, publiée en 2009, des biomarqueurs d'encéphalopathie néonatale est proposée par Murabayashi et al (Kinetics of serum S100B in newborns with intracranial lesions. Pediatr Int Off J Jpn Pediatr Soc 2008; 50(1):17-22*)*. Une autre méta-analyse a été publiée en 2009 par Ramaswamy et al (Systematic review of biomarkers of brain injury in term neonatal encephalopathy, Pediatric Neurology, 40, 2009, pp. 215-226*).*

Les méthodes de mesure utilisées pour le monitorage en continu de l'accouchement et les marqueurs utilisés ne permettent pas de détecter les risques de lésion cérébrale de manière satisfaisante.

L'invention vise à apporter une réponse pour l'évaluation du risque de santé, pour l'enfant, en particulier lors d'accouchements normaux et de nouveau-nés asymptomatiques.

L'invention s'appuie sur la découverte de ce que beaucoup d'accouchements par voie basse, considérés comme « normaux » peuvent entraîner des lésions cérébrales, sans modification de pH, avec un score Agpar normal, les nouveau-nés étant asymptomatiques, et que ces lésions pourraient être décelées par un écart de taux en protéine S100B par rapport à une valeur de sécurité, de préférence dans le sang ombilical.

Par protéine S100 B (ou S100β) on désigne ici une holoprotéine synthétisée essentiellement par les cellules gliales du système nerveux central et par les cellules de la gaine de Schwann. Cette protéine de 21kDa a été découverte en 1965 et est formée de deux sous-unités (deux unités béta ou une sous unité β et une sous unité α). Sa demi-vie plasmatique est de l'ordre de deux heures, et son élimination s'effectue par voie rénale. La protéine S100B est présente dans le cerveau de façon physiologique, et est libérée dans le liquide céphalorachidien et dans le sang en cas de lésion cérébrale. Il n'existe à ce jour aucune valeur de référence internationale, pour la concentration en protéine S100B dans le sérum.

Il est ainsi proposé, selon un premier aspect, l'utilisation de la protéine S100β dans l'identification de risques de lésions cérébrales, lors d'accouchements considérés comme normaux, chez des nouveaux nés asymptomatiques. En d'autres termes, l'invention propose la protéine S100B destinée à être utilisée dans l'identification de risques de lésions cérébrales, lors d'accouchements considérés comme normaux, chez les nouveau-nés asymptomatiques.

Selon le mode de réalisation, la protéine S100β est utilisée selon les dispositions suivantes,
- le dosage de la protéine S100β étant effectué dans le sang artériel ombilical ;
- une comparaison de la teneur en protéine S100β dans le sang artériel ombilical est effectuée, par rapport à une valeur seuil, la valeur seuil étant de 3.25 fois la valeur médiane.

L'invention se rapporte ainsi à une nouvelle utilisation d'une molécule connue, cette nouvelle utilisation facilitant l'identification de lésions cérébrales chez des nouveau-nés asymptomatiques, en particulier à l'issue d'accouchements considérés comme normaux.

La demanderesse a découvert que beaucoup d'accouchements par voie basse, considérés comme « normaux » (pH et score Apgar normaux, nouveau-né asymptomatique) peuvent entraîner des lésions cérébrales. Ces lésions pourraient être décelées par un écart de taux en protéine S100B par rapport à une valeur de sécurité, mesurée de préférence dans le sang, par exemple au niveau de la veine ou d'une artère du cordon ombilical, ou bien encore dans la salive, ou dans l'urine.

Les origines de ces phénomènes ne sont pas connues de la demanderesse qui propose plusieurs mécanismes.

En l'absence de souffrance fœtale ou d'anomalie pour le nouveau-né, aucune attention n'était jusqu'ici donnée au taux de protéine S100B.

Ainsi par exemple, le mode d'accouchement (voie basse ou césarienne) était considéré comme sans influence sur le taux de protéine S100B : en écartant les cas d'asphyxie périnatale et les cas de dystocie, Gazzolo et al n'observent aucune différence dans le taux de protéine S100B salivaire des nouveau-nés (S100B protein levels in saliva: correlation with gestational age in normal term and preterm newborns. Clin Biochem 2005;38(3):229-33*).*

De même, en ne prenant en compte que des accouchements spontanés par voie basse avec présentation céphalique et des césariennes, *Amer-Wåhlin et al* ne constatent aucune différence dans le taux de protéine S100B dans le sang ombilical, entre les nouveau-nés, et ne discernent aucune influence de la durée du travail, le taux élevé de protéine S100B chez le nouveau-né n'étant, pour ces auteurs, pas lié à un traumatisme ou une hypoxie pré ou périnatale, ce taux élevé étant observé chez le nouveau-né sain *(*Brain-specific NSE and S-100 proteins in umbilical blood after normal delivery. Clin Chim Acta Int J Clin Chem 2001;304(1-2):57-63*).*

Le dosage de la protéine S100 B a été proposé chez les nouveau-nés présentant des anomalies cérébrales ou des nouveaux nés à risque, ou bien encore dans le cas de grossesses à risque. La plus grande part des articles rapportant des résultats de recherche clinique sur ce thème sont issus de chercheurs italiens travaillant avec le Dr Diego Gazzolo. Le dosage de la protéine S100B a également été proposé chez des nouveau-né ayant subi une intervention chirurgicale.

Massaro et al ont présenté en 2014 une évaluation de la protéine 100B et de l'énolase neurospécifique (NSE) chez des nouveau-nés présentant une encéphalopathie et traités par hypothermie. Les 80 nouveau-nés pris en compte dans l'étude présentaient tous des signes marqués d'encéphalopathie, selon la classification de Sarnat, ainsi qu'une acidose et des faibles scores d'Apgar (Biomarkers S100B and neuron-specific enolase predict outcome in hypothermia-treated encephalopathic newborns. Pediatr Crit Care Med J Soc Crit Care Med World Fed Pediatr Intensive Crit Care Soc 2014;15(7):615-22)*.*

On peut se référer également aux documents suivants :
- pour le pronostic des nouveau-nés atteints d'encéphalopathies, avec traitement notamment par hypothermie : Massaro AN et al. Biomarkers of brain injury in neonatal encephalopathy treated with hypothermia. J Pediatr 2012;161(3):434-40 ; Çelik Y et al. The effects of selective head cooling versus whole-body cooling on some neural and inflammatory biomarkers: a randomized controlled pilot study. Ital J Pediatr 2015;41:79*;* Sun J et al Effects of hypothermia on NSE and S-100 protein levels in CSF in neonates following hypoxic/ischaemic brain damage. Acta Paediatr Oslo Nor 1992 2012;101(8):e316-20*;* Roka A et al Serum S100B and neuron-specific enolase levels in normothermic and hypothermic infants after perinatal asphyxia, Acta Paediatr Oslo Nor 1992 2012;101(3):319-23 ; Sofijanova A et al. Predicting outcome after severe brain injury in risk neonates using the serum S100B biomarker: results using single (24 h) time-point. Prilozi 2012; 33(1):147-56*;* Qian J et al Umbilical artery blood S100beta protein: a tool for the early identification of neonatal hypoxic-ischemic encephalopathy. Eur J Pediatr 2009;168(1):71-7 *;* Martins RO et al. S100B protein related neonatal hypoxia. Arq Neuropsiquiatr 2006;64(1):24-9 ; Gazzolo et al, S100B protein in urine of preterm newborns with ominous outcome, Pediatric research 58, 2005, pp. 1170-117 *;*
- pour le prognostic des prématurés: Serpero et al The clinical and diagnostic utility of S100B in preterm newborns, Clinica Chimica cta 444, 2015, pp. 193-198 *;* Sannia et al S100B urine concentrations in late preterm infants are gestational age and gender dependent. Clin Chim Acta Int J Clin Chem 2013; 417:31-4*;* Gazzolo et al S100B protein in urine of preterm newborns with ominous outcome. Pediatr Res 2005;58(6):1170-4*;*
- pour l'évaluation du risque de décès in-intéro: Florio et al Amniotic fluid S100B protein in mid-gestation and intrauterine fetal death, Lancet 2004, pp. 364-370*;*
- pour les accouchements avec acidose, détectée par mesure du pH du sang ombilical, et anomalie du rythme cardiaque, voir par exemple Stuart A et al. Fetal electrocardiographic monitoring during labor in relation to cord blood levels of the brain-injury marker protein S-100. J Perinat Med 2008;36(2):136-41*;* Kaandorp et al. Antenatal allopurinol for reduction of birth asphyxia induced brain damage (ALLO-Trial); a randomized double blind placebo controlled multicenter study. BMC Pregnancy Childbirth 2010;10:8 *;*
- pour le pronostic des nouveau-nés atteints de lésions intracrâniennes, décelées en tomographie ou IRM, voir par exemple Murabayashi M et al. Kinetics of serum S100B in newborns with intracranial lesions. Pediatr Int Off J Jpn Pediatr Soc 2008;50(1):17-22*;*
- pour le pronostic des nouveau-nés souffrants d'asphyxie et les risques de décès rapide, voir Gazzolo D et al. Diagnostic accuracy of S100B urinary testing at birth in full-term asphyxiated newborns to predict neonatal death. PIoS One 2009;4(2):e4298*.*

La protéine S100B a été abordée dans l'art antérieur comme un paramètre permettant d'évaluer la gravité de lésions cérébrales déjà constatées (par exemple par imagerie médicale, notamment IRM), ou très probables, les marqueurs habituels d'hypoxie étant anormaux, une valeur de référence étant choisie avec des nouveau-nés considérés comme sains, formant groupe de référence, ce groupe de référence étant défini à l'aide du score d'Apgar et du pH du sang ombilical, voir par exemple :
- Risso et al Perinatal asphyxia kidney failure does not affect S100B urine concentrations, Clinica Chimica 413, 2013, pp. 150-153 *;*
- Sannia et al, S100B protein maternal and foetal bloodstreams gradient in healthy and small for gestational age pregnancies, Clinica Chimica Acta, 412, 2011, pp. 1337-1340 *;*
- Sannia A, Risso FM, Serpero LD, et al. Antenatal glucocorticoid treatment affects preterm infants' S100B urine concentration in a dose-dependent manner, Clin Chim Acta Int J Clin Chem 2010;411(19-20):1539-41 *;*
- Risso FM, Serpero LD, Zimmermann LJI, et al. Urine S100 BB and A1B dimers are valuable predictors of adverse outcome in full-term asphyxiated infants, Acta Paediatr Oslo Nor 1992 2013; 102(10):e467-72*.*
- Maschmann J, Erb null, Heinemann MK, Ziemer G, Speer CP. Evaluation of protein S-100 serum concentrations in healthy newborns and seven newborns with perinatal acidosis, Acta Paediatr Oslo Nor 1992 2000; 89(5):553-5*.*

L'invention propose une approche différente, dans laquelle le taux de protéine S100B est systématiquement mesuré, un taux supérieur à une valeur seuil étant considéré comme significatif d'un risque de lésion cérébrale, même en cas d'accouchement normal et en présence d'un nouveau-né asymptomatique.

La demanderesse fait l'hypothèse que les accouchements normaux donnent lieu à des contraintes mécaniques élevées sur le crâne, et que ces contraintes sont devenues plus importantes dans les sociétés occidentales, entre autre par suite d'une augmentation des dimensions du crâne.

La demanderesse fait notamment l'hypothèse que l'augmentation des dimensions du crâne provient notamment d'un recours plus important aux césariennes. Une telle hypothèse a été proposée récemment par certains auteurs (cf. Mitteroecker P et al (2016) Cliff-edge model of obstetric selection in humans, Proc Natl Acad Sciences, 113(51):14680-14685*).*

La demanderesse fait l'hypothèse que lors d'un accouchement normal, les nouveau-nés subissent une compression cérébrale, souvent liée à un modelage temporaire des os du crâne du fœtus pendant le passage de la filière pelvi-génitale au niveau du détroit moyen. La demanderesse a établi qu'une telle compression cérébrale peut être déduite de mesures effectuées lors d'accouchements sous IRM, les images alimentant un modèle mécanique.

La demanderesse estime que cette compression cérébrale peut être responsable d'une contusion cérébrale qui pourrait être reflétée par l'élévation de la protéine S100β dans le sang.

La mesure de la concentration en protéine S100β dans le sang du cordon du nouveau-né, à titre systématique après un accouchement, permet une prédiction des risques d'hémorragies cérébrales et des risques d'ischémie, chez des nouveau-nés asymptomatiques.

Le dosage de la protéine S100β peut être effectué à l'aide de techniques de mesures commercialisées par différents fabricants (Sangtec, Elecsys Roche, CanAg, YK150). A titre d'exemple, il est possible d'utiliser le test Elecsys S100β, test immunologique pour la détermination quantitative in vitro de la protéine S100 (S100A1B et S100 BB) dans le sérum humain, ce test par électroluminescence ECLIA étant utilisé sur des analyseurs Elecsys et Cobas, ce test étant fabriqué par la société Roche Diagnostics Gmbh, et distribué par Roche Diagnostics France, le temps de dosage par ce test étant d'environ 18 minutes, le dosage étant peu couteux (coté BHN 120, 32€).

L'invention offre un moyen d'aide à la détection de lésions cérébrales à bas bruit, chez le nouveau-né asymptomatique, en particulier à l'issue d'accouchement considéré comme normal, aucun des moyens conventionnel (pH, lactase, RCF, score Apgar) ne classant le nouveau-né dans une catégorie considérée comme à risque.

L'invention permet ainsi un traitement précoce de lésions cérébrales ne donnant lieu qu'à des états inflammatoire à bas grade, le traitement permettant d'éviter les effets délétères de ces lésions sur le développement de l'enfant.

## Revendications

1. Utilisation de la protéine S100β dans l'identification de risques de lésions cérébrales (hémorragies, ischémies cérébrales), lors d'accouchements considérés comme normaux (accouchement eutocique), chez des nouveaux nés asymptomatiques, en l'absence de souffrance fœtale, le nouveau-né ne présentant aucun signe d'acidose ou d'hypoxie, et présentant un score d'Apgar normal, un pH de sang ombilical normal, les risques de lésions cérébrales étant identifiées par un taux de protéine S100β dans le sang artériel ombilical supérieur à une valeur seuil de sécurité, de 3.25 fois la valeur médiane.

## Patentansprüche

1. Verwendung des Proteins S100β zur Identifizierung des Risikos von Hirnschäden (Blutungen, Hirnischämien) bei als normal angesehenen Geburten (Spontangeburt) bei asymptomatischen Neugeborenen, wenn kein fetales Leiden vorliegt, das Neugeborene keine Anzeichen von Azidose oder Hypoxie aufweist und einen normalen Apgar-Score, einen normalen Nabelblut-pH-Wert aufweist, wobei das Risiko von Hirnschäden durch einen Spiegel des S100β-Proteins im arteriellen Nabelblut identifiziert wird, der über einem Sicherheitsgrenzwert des 3,25-fachen des Medianwerts liegt.

## Claims

1. A use of the S100β protein in the identification of risks of brain injuries (haemorrhages, brain ischemias), during deliveries considered normal (eutocic delivery), in asymptomatic newborns, in the absence of foetal suffering, the newborn showing no signs of acidosis or hypoxia, and having a normal Apgar score, normal umbilical blood pH, the risks of brain injuries being identified by a level of S100β protein in umbilical arterial blood above a safety threshold value of 3.25 times the median value.
